# EUROPEAN PATENT SPECIFICATION

(11) **EP 3 911 142 B1**
(45) Date of publication and mention of the grant of the patent: **24.12.2025**
(21) Application number: 20741567.0
(22) Date of filing: 14.01.2020
(51) Int. Cl.: A01D 46/00, A01D 45/00, A01D 46/30, A01D 91/04, A23N 15/00

(54) **SAFFRON COLLECTING DEVICE**
SAFRANSAMMELVORRICHTUNG
DISPOSITIF DE COLLECTE DE SAFRAN

(30) Priority: 15.01.2019 SE 1950037
(43) Date of publication of application: 24.11.2021
(73) Proprietor: Storp AB, 217 74 Malmö (SE)
(72) Inventor: MAHDJOUBI NAMIN, Amir Mehrdad, 211 34 MALMÖ (SE)
(74) Representative: AWA Sweden AB
(86) International application number: PCT/SE2020/050028
(87) International publication number: WO 2020/149779

(56) References cited:
- WO-A1-2018/220465
- CN-A- 107 258 236
- CN-A- 107 637 279
- CN-A- 107 637 279
- CN-U- 206 461 986
- CN-U- 208 191 360
- ES-A6- 2 006 024
- ES-U- 1 071 910
- ES-U- 1 071 910
- JP-A- 2018 121 563
- ASIMOPOULOS, N. ET AL.: "Autonomous Vehicle for Saffron Harvesting", PROCEDIA TECHNOLOGY, vol. 8, 2013, pages 175 - 182, XP055726266, ISSN: 2212-0173
- ANTONELLI, M.G.: "Development of a New Harvesting Module for Saffron Flower Detachment", ROMANIAN REVIEW PRECISION MECHANICS, OPTICS AND MECHATRONICS, vol. 39, 2011, pages 163 - 168, XP055726271, ISSN: 1584-5982
- GRACIA, L. ET AL.: "Automated cutting system to obtain the stigmas of the saffron flower", BIOSYSTEMS ENGINEERING, vol. 104, no. 1, 2009, pages 8 - 17, XP026460331, ISSN: 1537-5110, DOI: 10.1016/j.biosystemseng.2009.06.003

## Description

### Field of the invention

The present invention relates to a saffron collecting device and to a method for identifying and collecting pistils of saffron flowers.

### Technical background

There are some existing devices intended to simplify the harvesting of saffron. As an example, in CN107278513 there is disclosed an air-suction brush type saffron harvesting device. The saffron harvesting device is said to enable to achieve filament feeding and mechanical harvesting of the saffron seed balls in a natural growth state, and the working efficiency is said to be improved, and labor is liberated. Moreover, in CN107306592 there is disclosed a similar saffron harvesting device. Furthermore, in CN107660380 there is disclosed a pneumatic type saffron filament collection system. Also in this case there is a negative-pressure fan to enable automatic feeding of filaments on saffron seed balls.

Moreover, CN 107 258 236 A discloses a saffron collecting device according to the preamble of claim 1.

The present invention is directed to the provision of an optimal saffron harvesting and collecting device which reduces the labor needed, but which at the same type provides a high yield and high flexibility.

### Summary of the invention

The stated purpose above is achieved by a saffron collecting device comprising
- a sensor unit arranged for identifying pistils of saffron flowers;
- a pick unit arranged for removal of the pistils or part thereof from the saffron flowers when the sensor unit has identified the location of the pistils; and
- a collector unit arranged for collecting the pistils or part thereof in a collecting space,
wherein the pick unit is a suction unit and wherein the saffron collecting device also comprises a clip unit arranged to press the suction unit together.

The saffron collecting device operates by use of a sensor unit for the identifying of the pistils of the saffron flowers. Already in this regard, the saffron collecting device according to the present invention differs substantially from the ones disclosed in CN107278513, CN107306592 and CN107660380. The device according to the present invention is first and foremost directed to identifying the pistils of the saffron flowers, and subsequently to that to enable a simple and effective collecting principle of these pistils.

Moreover, in relation to the expression "pistil" it should be understood that this also embodies alternatives to this expression, such as "stigma (of the pistil)" or "stamen".

### Specific embodiments of the invention

Below some specific embodiment of the present invention are disclosed.

According to one embodiment, the collector unit is a bag. The type of bag may vary, but e.g. the type used as dust bags is one possible alternative.

According to one specific embodiment of the present invention, the sensor unit is a camera, IR sensor or radar sensor. Also combinations are possible according to the present invention. Moreover, also combinations of different sensors are possible according to the present invention. According to one preferred embodiment of the present invention, the sensor unit is a camera. As is clear from below, the saffron collecting device according to the present invention may comprise at least two camera units. Therefore, according to one specific embodiment of the present invention, the saffron collecting device comprises at least two sensor units, such as e.g. at least two camera units.

Moreover, according to one embodiment of the present invention, the saffron collecting device comprises at least one sensor unit, preferably a camera unit, for flower detection, and at least one sensor unit, preferably a camera unit, for detection of pistil(s) within the saffron flower. One such example is further explained below.

In this regard, the expression camera embodies any type of camera, such as a CCD (charge-coupled device) or a digital camera. Furthermore, the saffron collecting device according to the present invention may comprise several camera units and sensors, such as one camera unit, e.g. a multi-mono camera unit, for ensuring tracking to find a flower, and one or more camera units, e.g. a stereo pair camera unit, for the tracking within a specific saffron flower to find the pistils / stamens / stigmas of that flower. To give one example, a multi-mono camera unit may be arranged in a robot arm to ensure a suitable tracking and movement of the pick unit to the intended flower, and the pair of stereo cameras may be arranged in close proximity or at the pick unit to ensure efficient picking of the pistil or part of the pistil.

Furthermore, as may be understood from below, the sensor unit suitably comprises a sensor that detects color (reflecting light), physical shape (color separation) with visible light or with other electromagnetic radiation.

In CN107637279 there is disclosed a safflower picking robot. The present invention provides a device with, inter alia, another type of set-up in relation to the implemented sensor(s) and camera(s). Furthermore, the actual tracking technology used, suitably based on color, and the picking sequence, e.g. as explained below, and technology therefore also differ when comparing the present invention with CN107637279. These and other differences will become clearer when reviewing the description below.

Furthermore, according to the present invention, the pick unit is a suction unit, such as a filament suction unit. This may function in different ways, but a negative air pressure is suitable as an operation principle. It should, however, in this regard be noted that also using a negative air pressure in harvesting or a positive air pressure in releasing to a storage container is totally possible according to the present invention.

According to the present invention, negative air pressure does not have to be the only means to remove and collect the pistils once they have been identified. According to the present invention, the saffron collecting device also comprises a clip unit arranged to press the suction unit together. The clip unit may be arranged at different places in the device, and may also be part of the suction unit. According to one specific embodiment, the clip unit is arranged onto the suction unit and is programmed to press the same together once a pistil is located inside of the suction unit. The pistil is then fixated securely inside of the suction unit. By moving the suction unit upwards, the pistil is then loosened from the saffron flower and the negative air pressure in the suction unit enables the pistil to be sucked into the collector unit. This is further disclosed below in reference to the method according to the present invention.

It is of course possible, but not within the scope of the present invention, to work only based on negative air pressure or vacuum, i.e. without a clip unit. In such a case, however, there is a risk that the vacuum has to be so strong so that also other items may be sucked up into the suction unit. The incorporation of a clip unit avoids such a potential problem. Moreover, the suction unit may e.g. be made of a compressible material, e.g. a silicone material. This is also suitable so that a regular clip is enabled to press together the suction unit.

According to yet another specific embodiment, the pick unit and the sensor unit are integrated in a 3D unit. Such a 3D unit can be of different type, but may be a device that moves the suction unit and/or camera unit, e.g. one or more digital cameras, around in a harvesting area. For instance, a principle such as a 3D printer may be used also according to the present invention. As mentioned above, the sensor unit may be a camera. In line with this, according to on specific embodiment of the present invention, the sensor unit is a camera unit programmed to identify the pistils based on color. As the pistils have a distinct red color and the flowers are purple, the camera unit may be programmed to identify the pistils based on color and then enable the selection of these pistils to be picked by the pick unit.

One possible configuration of a 3D unit with camera unit(s) are discussed below in terms of functionality. The 3D technology enables the device to localize items in the 3D room, which in itself may be combined with the possibility of moving around in itself (consider mobile version of the device vs a fixed device). One or more camera units working in the visible light range enable to find and localize the pistils based on color. By using digital color filters and optical image processing the camera unit(s) can localize pistils. Moreover, according to one specific embodiment at least two camera sensors may be incorporated in the device according to the present invention. The two units may then operate together based on stereo positioning and triangulation. The position of each camera unit may then be calibrated. Images may then be used to further iterate the procedure until one specific pistil is located. As color filters are set, the entire procedure iterates until the specific color or color range of pistils of saffron flowers are detected.

Based on what has been described above, the area of color filtration and detection may be incorporated into the present invention independently on the camera configuration. Moreover, possible stereo and triangulation methods used may be set according to regular standards. The possible specific adjustment in this technical application may be set into the specific algorithm based on the intended target, i.e. pistils in saffron flowers. This implies that the programming incorporated into the control system of the saffron collecting device should be adapted in accordance with this intended target area.

As may be understood from above, the saffron collecting device suitably operates by means of a processor unit. The technology type may differ, but examples are where the processor unit is a cartesian product or a delta product, e.g. a delta servo product. A delta type is preferred according to the present invention.

The saffron collecting device according to the present invention may be a fixed system or a mobile system. It should, however, be noted that the present invention is in particular related to a mobile system, such as an automated device, such as a robotic device. In the case of a fixed system then saffron flowers have to be harvested and collected first and then emptied or fed to the saffron collecting device according to the present invention. With a mobile saffron collecting device according to the present invention it is possible to obtain the saffron pistils directly from the flowers without first harvesting the same. As may be understood, the mobile saffron collecting device according to the present invention is preferred. In this case, the device performs the entire operation directly on the flowers in an automatic way, without the need for a harvesting operation first. In such a case, one may say that the saffron collecting device also operates as a saffron harvesting and collecting device.

In relation to harvesting and the present invention it may also be said that when only harvesting the pistils the amount of biomass that are removed is relatively small and therefore the need of added fertilizers is low.

In the case of a fixed system, then the saffron collecting device may be arranged in connection to a feeding belt intended for saffron flowers. As such, harvested flowers may be directly put onto this feeding belt.

A mobile device according to the present invention may, with reference to its propulsion principle, be compared to a robotic lawn-mover. The device according to the present invention must also be able to identify the pistils of the saffron flowers. In line with the general description above, according to one specific embodiment of the present invention, the saffron collecting device comprises an automatic propulsion unit and thus is a saffron harvesting and collecting robotic device. Furthermore, according to yet another specific embodiment, the saffron harvesting and collecting robotic device comprises propulsion belts. Moreover, according to one specific embodiment of the present invention, the saffron harvesting and collecting robotic device is arranged with an open space for the sensor unit, such as a camera, and collector unit to be able to detect and pick pistils in growing saffron flowers.

The present invention is also directed to a method for identifying and collecting pistils of saffron flowers. Therefore, according to one embodiment, the present invention refers to a method for identifying and collecting pistils of saffron flowers, wherein a saffron collecting device according to the present invention performs the following operations:
- locating a pistil of a saffron flower by means of the sensor unit;
- directing a pick unit to the pistil;
- removing the pistil from the saffron flower by means of the pick unit; and
- directing the pick unit to drop or send the pistil for collecting the pistil in the collector unit,
wherein the pick unit is a suction unit and where an open end part of the suction unit encloses at least part of the pistil, a clip unit is arranged to then squeeze together said end part of the suction unit to fixate the suction unit around the pistil in a tight way, the suction unit is then directed upwards to loosen or pull the pistil up from the saffron flower and finally the clip unit releases and a negative air pressure allows the suction unit to suck the pistil into the collector unit for collecting the same in the collecting space.

The operation as such in terms of detection of pistils has been discussed above in general terms.

As said, according to the present invention, the pick unit is a suction unit and where an open end part of the suction unit encloses at least part of the pistil, a clip unit is arranged to then squeeze together said end part of the suction unit to fixate the suction unit around the pistil in a tight way, the suction unit is then directed upwards to loosen or pull the pistil up from the saffron flower and finally the clip unit releases and a negative air pressure allows the suction unit to suck the pistil into the collector unit for collecting the same in the collecting space. As may be understood from above, according to this embodiment the pistil is already loosened when it is finally sucked up into the collector unit. This further implies that the saffron collecting device may have a set negative air pressure which is enough to suck such fixated and loosened pistils, however not loosen the pistils from the flower as such.

It should be noted that the sequence of the method may be performed in different ways. The following is another example how the procedure may be performed. First the camera unit identifies saffron flowers and provides X, Y and Z coordinates to each flower cluster inside of the working area of the picking area. The X coordinate is changed when the robot device moves in one direction. When the X coordinate is the same as the center of the flower cluster, then the pick unit is moved to the right Y coordinate. As mentioned below, the sensor unit may comprise several camera units. For instance, a single or double stereo camera units may take over the next step in the operation sequence once the right y coordinate has been set. The stereo cameras then control the pick unit to be driven to the top of a pistil of the saffron flower. This procedure may be accomplished based on color identification in the software used. The procedure above may be seen as an effective way of ensuring that the pick unit is ensured to be arranged in the right place before starting the actual picking procedure.

As a next step, the pick unit is opened and vacuum is provided so that the pick unit is in a sucking mode. The pick unit then is moved to the root of the pistil and is closed. The pick unit is then moved in a Z coordinate direction to loosen the pistil. Once the movement is finalized then the pick unit releases its grip of the pistil. The vacuum provided ensures that the pistil is sucked into the collector unit, such as a bag or container or the like. The pick unit moves to the next pistil top and at the same time the pick unit e.g. closes once the earlier pistil is detected in the collector unit. Repetition is performed of the actual picking sequence until all pistils in the flower cluster has been picked. Then movement is made to the next flower cluster, such as described in the beginning of the sequence.

It should be noted that the above is explaining a sequence in a dry condition. In wet, mist or rain, a pick and place method is needed. Suction to harvest and a blow out when a pick unit head has moved to the collecting point are suitably incorporated.

### Detailed description of the drawings

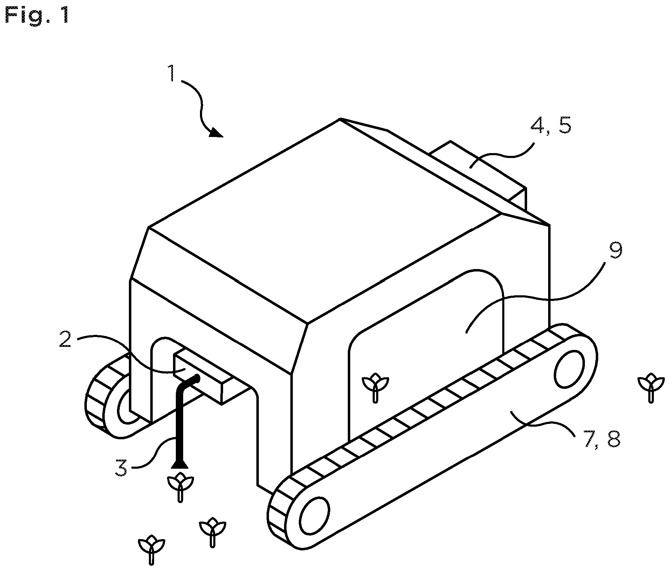

In fig. 1 there is shown an example useful for understanding the invention. In this case the saffron collecting device 1 is in the form of a mobile version, such as a robotic self-transporting saffron collecting device 1. The saffron collecting device 1 comprises a sensor unit 2, which suitably comprises one or more camera units. Moreover, the saffron collecting device 1 also comprises a pick unit arranged for removal of the pistils or part thereof from the saffron flowers when the sensor unit 2 has identified the location of the pistils. In this case the sensor unit 2 and the pick unit 3 are integrated in one and the same 3D unit. Moreover, in this case the pick unit 3 is in the form of a suction unit which enables to suck up the pistils of the saffron flowers. A collector unit 4 is arranged for collecting the pistils or part thereof in a collecting space 5. This may be a box which comprises a bag being connected to the suction unit, similar to the concept of a vacuum cleaner. Furthermore, according to this embodiment, the saffron collecting device 1 comprises an automatic propulsion unit 7, i.e. the device 1 is a saffron harvesting and collecting robotic device, with propulsion belts 8 in this case. Furthermore, the saffron harvesting and collecting robotic device 1 is arranged with an open space 9 for the camera unit and collector unit to be able to detect and pick pistils in growing saffron flowers in an effective way.

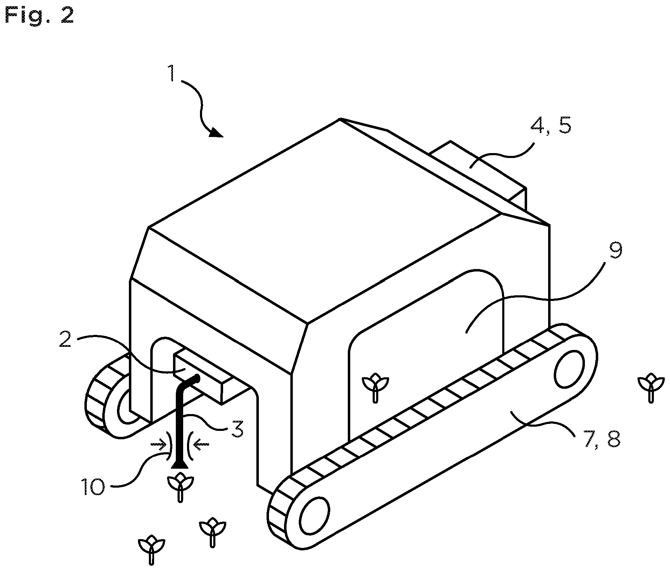

In fig. 2 there is shown an embodiment of the invention, which is similar to the device shown in fig. 1, however in this case the device 1 also comprises a clip unit 10. This clip unit 10 is part of the 3D unit with sensor unit and pick unit, i.e. suction unit. The clip is arranged to enable to squeeze the suction unit so that an enclosed pistil may be pulled up and loosened from a saffron flower.

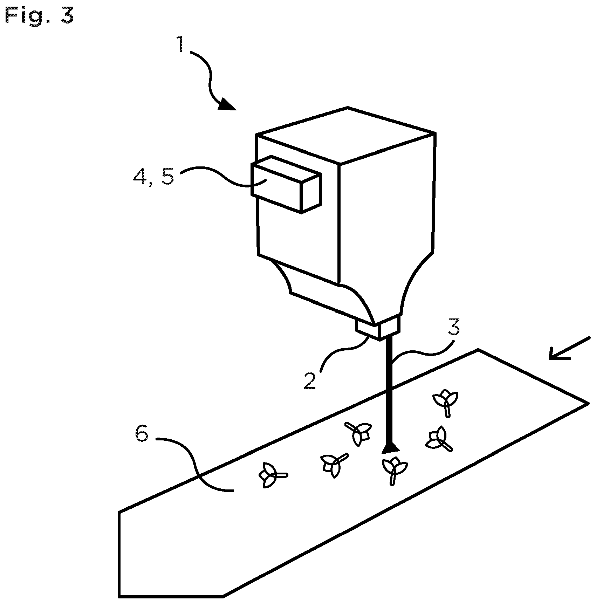

In fig. 3 there is shown another example, which, per se, does not fall within the scope of the present invention. In this case, the device 1 is a fixed device. Therefore, the saffron collecting device 1 is arranged in connection to a feeding belt 6 intended for saffron flowers. Otherwise, also in this case the saffron collecting device 1 comprises a sensor unit 2, which suitably comprises one or more camera units. Moreover, the saffron collecting device 1 also comprises a pick unit arranged for removal of the pistils or part thereof from the saffron flowers when the sensor unit 2 has identified the location of the pistils. Also in this case the sensor unit 2 and the pick unit 3 are integrated in one and the same 3D unit. Moreover, also in this case the pick unit 3 is in the form of a suction unit which enables to suck up the pistils of the saffron flowers and collect the pistils in a collector unit 4 connected to a collecting space 5.

## Claims

1. A saffron collecting device (1) comprising
- a sensor unit (2) arranged for identifying pistils of saffron flowers;
- a pick unit (3) arranged for removal of the pistils or part thereof from the saffron flowers when the sensor unit (2) has identified the location of the pistils; and
- a collector unit (4) arranged for collecting the pistils or part thereof in a collecting space (5),
wherein the pick unit (3) is a suction unit; **characterised in that** the saffron collecting device (1) also comprises a clip unit (10) arranged to press the suction unit together.

2. The saffron collecting device (1) according to claim 1, wherein the sensor unit (2) is a camera, IR sensor or radar sensor, preferably a camera.

3. The saffron collecting device (1) according to claim 1 or 2, wherein the saffron collecting device (1) comprises at least two sensor units (2).

4. The saffron collecting device (1) according to any of claims 1-3, wherein the saffron collecting device (1) comprises at least one sensor unit for flower detection, and at least one sensor unit for detection of pistil(s) within the saffron flower.

5. The saffron collecting device (1) according to any of claims 1-4, wherein the pick unit (3) and the sensor unit (2) are integrated in a 3D unit.

6. The saffron collecting device (1) according to any of claims 1-5, wherein the sensor unit (2) is a camera unit programmed to identify the pistils based on color.

7. The saffron collecting device (1) according to any of claims 1-6, wherein the saffron collecting device (1) operates by means of a processor unit.

8. The saffron collecting device (1) according to claim 7, wherein the processor unit is a cartesian product or a delta servo product.

9. The saffron collecting device (1) according to any of claims 1-8, wherein the saffron collecting device (1) comprises an automatic propulsion unit (7) and thus is a saffron harvesting and collecting robotic device.

10. The saffron collecting device (1) according to claim 9, wherein the saffron harvesting and collecting robotic device is arranged with an open space (9) for the sensor unit (2) and collector unit (4) to be able to detect and pick pistils in growing saffron flowers.

11. A method for identifying and collecting pistils of saffron flowers, wherein a saffron collecting device (1) according to any of claims 1-10 performs the following operations:
- locating a pistil of a saffron flower by means of the sensor unit (2);
- directing a pick unit (3) to the pistil;
- removing the pistil from the saffron flower by means of the pick unit (3); and
- directing the pick unit (3) to drop or send the pistil for collecting the pistil in the collector unit (4), wherein the pick unit (3) is a suction unit and wherein an open end part of the suction unit (3) encloses at least part of the pistil, a clip unit (10) is arranged to then squeeze together said end part of the suction unit (3) to fixate the suction unit (3) around the pistil in a tight way, the suction unit (3) is then directed upwards to loosen or pull the pistil up from the saffron flower and finally the clip unit releases and a negative air pressure allows the suction unit (3) to suck the pistil into the collecting unit (4) for collecting the same in the collecting space (5).

## Patentansprüche

1. Safransammelvorrichtung (1), umfassend:
- eine Sensoreinheit (2), die zum Identifizieren von Stempeln von Safranblüten angeordnet sind;
- eine Pflückeinheit (3), die zum Entnehmen der Stempel oder eines Teils davon aus den Safranblüten angeordnet ist, wenn die Sensoreinheit (2) die Position der Stempel identifiziert hat; und
- eine Sammlereinheit (4), die zum Sammeln der Stempel oder des Teils davon in einem Sammelraum (5) angeordnet ist,
wobei die Pflückeinheit (3) eine Saugeinheit ist; **dadurch gekennzeichnet, dass** die Safransammelvorrichtung (1) außerdem eine Klammereinheit (10) umfasst, die zum Zusammenpressen der Saugeinheit angeordnet ist.

2. Safransammelvorrichtung (1) nach Anspruch 1, wobei die Sensoreinheit (2) eine Kamera, ein IR-Sensor oder ein Radarsensor, vorzugsweise eine Kamera, ist.

3. Safransammelvorrichtung (1) nach Anspruch 1 oder 2, wobei die Safransammelvorrichtung (1) mindestens zwei Sensoreinheiten (2) umfasst.

4. Safransammelvorrichtung (1) nach einem der Ansprüche 1-3, wobei die Safransammelvorrichtung (1) mindestens eine Sensoreinheit zur Blütenerkennung und mindestens eine Sensoreinheit zum Erkennen des/der Stempel in der Safranblüte umfasst.

5. Safransammelvorrichtung (1) nach einem der Ansprüche 1-4, wobei die Pflückeinheit (3) und die Sensoreinheit (2) in einer 3D-Einheit integriert sind.

6. Safransammelvorrichtung (1) nach einem der Ansprüche 1-5, wobei die Sensoreinheit (2) eine Kameraeinheit ist, die programmiert ist, die Stempel auf der Grundlage von Farbe zu identifizieren.

7. Safransammelvorrichtung (1) nach einem der Ansprüche 1-6, wobei die Safransammelvorrichtung (1) mittels einer Prozessoreinheit arbeitet.

8. Safransammelvorrichtung (1) nach Anspruch 7, wobei die Prozessoreinheit ein kartesisches Produkt oder ein Delta-Servo-Produkt ist.

9. Safransammelvorrichtung (1) nach einem der Ansprüche 1-8, wobei die Safransammelvorrichtung (1) eine automatische Vorschubeinheit (7) umfasst und somit eine Safranernte- und -sammelrobotervorrichtung ist.

10. Safransammelvorrichtung (1) nach Anspruch 9, wobei die Safranernte- und -sammelrobotervorrichtung mit einem offenen Raum (9) angeordnet ist, damit die Sensoreinheit (2) und die Sammlereinheit (4) in der Lage sind, Stempel in wachsenden Safranblüten zu erkennen und zu pflücken.

11. Verfahren zum Identifizieren und Sammeln von Stempeln von Safranblüten, wobei die Safransammelvorrichtung (1) nach einem der Ansprüche 1-10 die folgenden Operationen durchführt:
- Bestimmen der Position einer Safranblüte mittels der Sensoreinheit (2);
- Steuern einer Pflückeinheit (3) zu dem Stempel;
- Entnehmen des Stempels aus der Safranblüte mittels der Pflückeinheit (3); und
- Steuern der Pflückeinheit (3), den Stempel zum Sammeln des Stempels in die Sammlereinheit (4) zu legen oder zu senden, wobei die Pflückeinheit (3) eine Saugeinheit ist und wobei ein offener Endteil der Saugeinheit (3) mindestens einen Teil des Stempels umschließt, eine Klammereinheit (10) angeordnet ist, den Endteil der Saugeinheit (3) dann zusammenzupressen, um die Saugeinheit (3) fest um den Stempel zu fixieren, die Saugeinheit (3) dann nach oben gesteuert wird, um den Stempel aus der Safranblüte zu lockern oder zu ziehen, und sich schließlich die Klammereinheit öffnet und ein Luftunterdruck es der Saugeinheit (3) ermöglicht, den Stempel in die Sammeleinheit (4) zu saugen, um ihn in dem Sammelraum (5) zu sammeln.

## Revendications

1. Dispositif de collecte de safran (1) comprenant
- une unité de détection (2) agencée pour identifier les pistils de fleurs de safran ;
- une unité de saisie (3) agencée pour enlever les pistils ou une partie de ceux-ci des fleurs de safran lorsque l'unité de détection (2) a identifié l'emplacement des pistils ; et
- une unité de collecte (4) agencée pour collecter les pistils, ou une partie de ceux-ci dans un espace de collecte (5),
dans lequel l'unité de saisie (3) est une unité d'aspiration ; **caractérisé en ce que** le dispositif de collecte de safran (1) comprend également une unité de pince (10) agencée pour resserrer l'unité d'aspiration.

2. Dispositif de collecte de safran (1) selon la revendication 1, dans lequel l'unité de détection (2) est une caméra, un capteur IR ou un capteur radar, de préférence une caméra.

3. Dispositif de collecte de safran (1) selon la revendication 1 ou 2, dans lequel le dispositif de collecte de safran (1) comprend au moins deux unités de détection (2).

4. Dispositif de collecte de safran (1) selon l'une quelconque des revendications 1 à 3, dans lequel le dispositif de collecte de safran (1) comprend au moins une unité de détection pour la détection de fleurs, et au moins une unité de détection pour la détection de pistil(s) à l'intérieur de la fleur de safran.

5. Dispositif de collecte de safran (1) selon l'une quelconque des revendications 1 à 4, dans lequel l'unité de saisie (3) et l'unité de détection (2) sont intégrées dans une unité 3D.

6. Dispositif de collecte de safran (1) selon l'une quelconque des revendications 1 à 5, dans lequel l'unité de détection (2) est une unité de caméra programmée pour identifier les pistils sur la base de la couleur.

7. Dispositif de collecte de safran (1) selon l'une quelconque des revendications 1 à 6, dans lequel le dispositif de collecte de safran (1) fonctionne au moyen d'une unité de processeur.

8. Dispositif de collecte de safran (1) selon la revendication 7, dans lequel l'unité de processeur est un produit cartésien ou un servo-produit delta.

9. Dispositif de collecte de safran (1) selon l'une quelconque des revendications 1 à 8, dans lequel le dispositif de collecte de safran (1) comprend une unité de propulsion automatique (7) et est donc un dispositif robotique de récolte et de collecte de safran.

10. Dispositif de collecte de safran (1) selon la revendication 9, dans lequel le dispositif robotique de récolte et de collecte de safran est muni d'un espace ouvert (9) pour que l'unité de détection (2) et l'unité de collecte (4) puissent détecter et saisir des pistils dans les fleurs de safran cultivées.

11. Procédé d'identification et de collecte de pistils de fleurs de safran, dans lequel un dispositif de collecte de safran (1) selon l'une quelconque des revendications 1 à 10 effectue les opérations suivantes :
- la localisation d'un pistil d'une fleur de safran au moyen de l'unité de détection (2) ;
- l'orientation d'une unité de saisie (3) vers le pistil ;
- le retrait du pistil de la fleur de safran au moyen de l'unité de saisie (3) ; et
- l'orientation de l'unité de saisie (3) de façon à faire tomber ou envoyer le pistil pour collecter le pistil dans l'unité de collecte (4), l'unité de saisie (3) étant une unité d'aspiration et une partie d'extrémité ouverte de l'unité d'aspiration (3) enfermant au moins une partie du pistil, dans lequel une unité de pince (10) est agencée pour ensuite resserrer ladite partie d'extrémité de l'unité d'aspiration (3) pour fixer de manière serrée l'unité d'aspiration (3) autour du pistil, l'unité d'aspiration (3) est ensuite orientée vers le haut pour desserrer ou tirer le pistil vers le haut depuis la fleur de safran et enfin l'unité de pince se libère et une pression d'air négative permet à l'unité d'aspiration (3) d'aspirer le pistil dans l'unité de collecte (4) pour collecter celui-ci dans l'espace de collecte (5).
